# FASCICULE DE BREVET EUROPEEN

(11) **EP 1 252 882 B1**
(45) Date de publication et mention de la délivrance du brevet: **21.03.2007**
(21) Numéro de dépôt: 02291030.1
(22) Date de dépôt: 23.04.2002
(51) Int. Cl.: A61K 8/44, A61K 31/198

(54) **Procede pour augmenter le seuil de tolerance d'une peau sensible ou intolerante**
Verfahren zur Erhöhung der Schwellentoleranz bei empfindlicher Haut
Process to increase the threshold tolerance of the sensitive skin

(30) Priorité: 23.04.2001 FR 0105457
(43) Date de publication de la demande: 30.10.2002
(73) Titulaire: L'ORÉAL, 75008 Paris (FR)
(72) Inventeur: De Lacharriere, Olivier, 75015 Paris (FR); Jourdain, Roland, 92360 Meudon la Foret (FR)
(74) Mandataire: Catherine, Alain

(56) Documents cités:
- EP-A- 0 650 720
- EP-A- 0 700 896
- EP-A- 0 856 308
- WO-A-90/14833
- WO-A-97/31620
- WO-A-98/01110
- US-A- 5 208 013
- PATENT ABSTRACTS OF JAPAN vol. 2000, no. 08, 6 octobre 2000 (2000-10-06) & JP 2000 136122 A (KOSE CORP), 16 mai 2000 (2000-05-16)
- DATABASE WPI Section Ch, Week 199814 Derwent Publications Ltd., London, GB; Class B05, AN 1998-148357 XP002193650 & JP 10 017493 A (FUJII M), 20 janvier 1998 (1998-01-20)

## Description

La présente invention se rapporte à un procédé pour traiter les peaux sensibles ou intolérantes permettant notamment d'augmenter le seuil de tolérance de la peau sensible ou intolérante, permettant ainsi de diminuer, voire de supprimer les signes sensoriels associés à la peau sensible ou intolérante.

Par peau, on entend au sens de la demande toute zone cutanée du corps, le cuir chevelu, ou les muqueuses (buccale, jugale, gingivale et conjonctive) de l'être humain.

Par seuil de tolérance de la peau, on entend au sens de la demande le seuil d'excitabilité de la peau au-delà duquel la peau répond à une agression externe par une hyperréactivité cutanée se manifestant par des signes sensoriels tels que picotements, échauffements, tiraillements, démangeaisons, sensations d'inconfort de la peau, associés ou non à des rougeurs cutanées.

Par agression externe, on entend aussi bien les produits à caractère irritant tels que les tensioactifs, les conservateurs, les parfums, le savon, l'eau dure à forte concentration de calcaire, la laine, que l'environnement, comme les variations de température et le vent, ou encore les frottements comme le feu du rasoir, etc, à l'exclusion toutefois des produits allergènes.

Dans le domaine des désordres cutanés, il est connu que la peau réagit aux agressions externes et que certaines peaux réagissent beaucoup plus rapidement à ces agressions et sont plus sensibles ou plus intolérantes qu'une peau normale.

Les mécanismes par lesquels la peau réagit à ces agressions étaient jusqu'à présent inconnus. Ainsi, par exemple, personne ne connaissait exactement le processus mis en cause dans la sensibilité ou l'intolérance de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Par ailleurs, on assimilait les peaux sensibles ou intolérantes à des peaux allergiques.

Du fait que l'on connaissait mal les mécanismes par lesquels la peau réagit aux agressions externes, il était jusqu'à présent très difficile de prévenir une réponse de la peau à ces agressions et particulièrement de traiter les peaux sensibles ou intolérantes; Ainsi, on les traitait indirectement, par exemple en limitant dans les compositions cosmétiques l'emploi de produits à caractère irritant, tels que les tensioactifs, les conservateurs ou les parfums.

Certains tests ont bien été mis au point pour tenter de cerner les peaux sensibles, par exemple des tests à l'acide lactique et au DMSO qui sont connus pour être des substances irritantes: voir par exemple l'article de L. Lammintausta et al., Dermatoses, 1998, 36, pages 45-49, et l'article de T. Agner et J. Serup. Clinical and Experimental Dermatology, 1989, 14, pages 214-217.

Malheureusement, ces tests ne permettaient pas de caractériser correctement les mécanismes par lesquels la peau réagit aux agressions externes, ni de comprendre ceux régissant les peaux sensibles ou les peaux intolérantes.

Il est aujourd'hui connu que la réaction de la peau aux agressions externes est liée à l'excitabilité des nerfs sensitifs cutanés. En outre, il est également connu que les peaux sensibles ou intolérantes, qui ne sont ni des inflammations, ni des peaux allergiques dans la mesure où aucun mécanisme immunologique n'est mis en oeuvre, répondent aux agressions externes par les mêmes signes que la peau normale mais de manière plus rapide et parfois plus violente.

Ainsi, la peau sensible ou intolérante se caractérise par un ensemble de signes dont sont clairement exclus les signes de l'inflammation, comme l'oedème, et/ou les signes de l'allergie, qui sont toujours accompagnés d'une réaction inflammatoire et donc d'un oedème.

Ainsi, la demande de brevet français FR-9710853 décrit l'utilisation d'une ou plusieurs substances agonistes pour activer les récepteurs des canaux chlore ou potassique des fibres nerveuses sensitives du système nerveux périphérique cutané pour le traitement des peaux sensibles. En outre, la demande de brevet français FR-9802783 décrit l'utilisation d'un composé inhibant l'activité des canaux sodique ou calcique des fibres nerveuses sensitives du système nerveux périphérique cutané pour augmenter le seuil de tolérance de la peau, et plus particulièrement de la peau sensible ou intolérante.

La demanderesse a maintenant découvert que l'application topique d'une composition contenant une quantité effective d'au moins un agent chélateur d'ions permettait d'augmenter le seuil de tolérance de la peau sensible ou intolérante et donc d'améliorer le confort cutané des individus à peau sensible.

Plus particulièrement, la demanderesse a trouvé que l'application topique d'une composition contenant une quantité effective d'EDTA permettait de diminuer l'hyperréactivité cutanée de sujets à peau sensible lors du test de picotements à l'acide lactique (Lactic Acid Stinging Test).

Le test de picotements à l'acide lactique, ou Lactic Acid Stinging Test, est un test d'évaluation de la réactivité cutanée chez les sujets bien caractérisés par ailleurs pour avoir une peau sensible. Le Lactic Acid Stinging test consiste à appliquer sur une zone de la peau située au niveau des sillons nasogéniens, une solution d'acide lactique, et à noter l'apparition et l'intensité des signes subjectifs provoqués par cette application, notamment des picotements.

L'homme de l'art connaît l'utilisation d'agents chélateurs dans des compositions pour une application topique sur la peau ou le cuir chevelu.

En effet, la demande internationale WO-9503032 décrit une composition pour traitement topique contenant des alpha-hydroxyacides (AHA) pour améliorer l'apparence de la peau ridée, pelée, âgée ou photoâgée. Des agents chélateurs, principalement des ions Zn²⁺ et Mg²⁺, sont utilisés pour améliorer l'efficacité des AHA par un effet synergique qui permet de réduire la concentration d'AHA. La diminution de l'irritation et des sensations de picotements est liée à la diminution de la concentration des AHA.

D'autre part, la demande de brevet français FR-9802130 décrit une composition cosmétique contenant des éléments traces issus d'eaux minérales et des agents chélateurs issus d'hydrolysats protéiques d'origine végétale, utilisée pour améliorer l'état et l'apparence de la peau, des cheveux et des ongles, stimuler le renouvellement cellulaire et combattre le vieillissement cutanée, et pour son activité photoprotectrice antibactérienne, antivirale, antifongicide, antiinflammatoire et anti-chute.

De même, le brevet européen EP-700896 décrit l'utilisation d'un agent chélateur des métaux de transition dans une composition cosmétique ou dermatologique pour protéger la peau, les cheveux et les muqueuses contre la lumière et le vieillissement.

Enfin, les brevets japonais JP-1049631 et JP-0182407 décrivent respectivement un produit cosmétique hydratant contenant un polymère et un agent chélateur pour l'amélioration de la peau sèche, et une composition topique contenant des extraits de momordical finctus et un agent chélateur du fer pour la prévention de l'inflammation, du vieillissement, du noircissement cutané et l'amélioration de la peau sèche.

Aucun de ces documents ne concerne l'utilisation d'agents chélateurs d'ions pour augmenter le seuil de tolérance des peaux sensibles ou intolérantes.

La demande internationale WO 90/14833 décrit des formulations aqueuses à base de rétinoïdes, destinées à une application topique sur la peau, permettant une libération lente de ces rétinoïdes et une faible irritation de la peau. Ces formulations peuvent également contenir des agents chélateurs, mais leur fonction est clairement définie comme étant une aide pour retenir les rétinoïdes (ou d'autres composés irritants) dans la composition, en vue de l'obtention d'une libération lente de ces derniers.

L'invention a donc pour objet l'utilisation pour augmenter le seuil de tolérance de la peau sensible ou intolérante, qui n'est pas une peau allergique, dans une composition cosmétique et/ou dermatologique et/ou de l'hygiène d'une quantité effective d'au moins un agent chélateur d'ions.

Par chélateurs d'ions, on entend les composés chimiques ou biologiques (protéines, peptides ...) ayant la capacité de perturber et de modifier l'environnement ionique péricellulaire au niveau des cellules cutanées en séquestrant certains ions, notamment les ions Na⁺, K⁺, Ca²⁺ et Cl⁻.

A titre d'exemples de chélateurs chimiques, on peut citer:
- l'acide aminotriméthyle phosphonique,
- l'acide β-alanine diacétique,
- l'acide citrique,
- la cyclodextrine,
- l'acide cyclohexanediamine tétracétique,
- l'acide diéthylènetriamine pentaméthylène phosphonique,
- l'acide diéthanolamine N-acétique (diéthanolamine N-acétique),
- l'acide éthylène diamine tétracétique (EDTA ou YH₄) et ses sels de sodium (YH₃Na, Y₂H₂Na₂, YHNa₃ et YNa₄), de potassium (YH₃K, Y₂H₃K₃ et YK₄), de calcium disodium, de diammonium et ses sels de triéthanolamine (TEA-EDTA),
- l'acide étidronique,
- l'acide galactanique,
- l'acide hydroxyéthyl éthylènediamine tétracétique (HEDTA) et son sel trisodique,
- l'acide gluconique,
- l'acide glucuronique,
- l'acide nitrilotriacétique (NTA) et son sel trisodique,
- l'acide pentétique,
- l'acide phytique,
- l'acide ribonique,
- le citrate de diammonium,
- l'azacycloheptane diphosphonate de disodium,
- le pyrophoshate de disodium,
- l'hydroxypropyl cyclodextrine,
- le méthyl cyclodextrine,
- le triphosphate de pentapotassium,
- l'aminotriméthylène phosphonate de pentasodium,
- l'éthylènediamine tétraméthylène phosphonate de pentasodium,
- le pentétate de pentasodium,
- le triphosphate de pentasodium,
- le citrate de potassium,
- EDTMP de potassium,
- EDTMP de sodium,
- le chitosan méthylène phosphonate de sodium,
- l'hexamétaphosphate de sodium,
- le métaphosphate de sodium,
- le polyphosphate de potassium,
- le polyphosphate de sodium,
- le trimétaphosphate de sodium,
- le dihydroxyéthylglycinate de sodium,
- le gluconate de potassium,
- le gluconate de sodium,
- le glucopeptate de sodium,
- le glycereth-1 polyphosphate de sodium,
- le pyrophosphate de tétrapotassium,
- le polyphosphate de triéthanolamine (TEA),
- le pyrophosphate de tétrasodium,
- le phosphate de trisodium,
- l'oxyde triphosphonométhylamine de potassium,
- le métasilicate de sodium,
- le phytate de sodium,
- le polydiméthylglycinophénolsulfonate de sodium,
- le tétrahydroxyéthyl éthylène diamine,
- le tétrahydroxypropyl éthylène diamine,
- l'étidronate de tétrapotassium,
- l'étidronate de tétrasodium,
- l'iminodisuccinate de tétrasodium,
- l'éthylènediamine disuccinate de trisodium,
- l'acide éthanolamine N,N-diacétique,
- l'acétate de disodium,
- le dimercaprol,
- la déferoxamine,
- le Zylox, chélateur du fer décrit et revendique dans la demande internationale WO 94/61338,
- sans que cette liste soit limitative.

A titre d'exemples de chélateurs biologiques, on peut citer, la métallothionéine, la transferrine, la calmoduline, et le chitosan méthylène phosphonate de sodium

Préférentiellement selon l'invention, on utilise un agent chélateur chimique choisi parmi l'acide éthylène diamine tétracétique (EDTA) et ses sels de sodium, potassium, calcium, disodium, diamonium, triéthanolamine (TEA-EDTA), l'acide hydroxyéthyl éthylène diamine tétracétique (HEDTA) et son sel trisodique, et leurs mélanges.

L'agent chélateur est présent dans la composition utilisée dans le procédé selon l'invention à raison de 10⁻⁶ à 10% en poids, et de préférence de 0,1 à 5% en poids, et mieux environ 2% en poids du poids total de la composition.

Les compositions utilisées dans le procédé selon l'invention peuvent en outre comporter au moins une phase grasse, liquide ou solide.

Par phase grasse liquide, au sens de la demande, on entend une phase grasse liquide à la température ambiante (25°C) composée d'un ou plusieurs corps gras liquides à température ambiante, également appelés huiles, compatibles entre eux.

Les huiles de la phase grasse des compositions de l'invention peuvent être des huiles, polaires ou non polaires, volatiles ou non volatiles, choisies parmi les huiles classiquement utilisées en cosmétique.

Parmi les huiles polaires, on peut citer les huiles hydrocarbonées comportant des fonctions esters, éthers, acides, alcools ou leurs mélanges, telles que par exemple:
- les huiles hydrocarbonées à forte teneur en triglycérides constitués d'ester d'acides gras et de glycérol, dont les acides gras peuvent avoir des longueurs de chaînes variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol 810, 812 et 818 par la société Dynamit Nobel,
- les huiles de synthèse de formule R¹COOR² dans laquelle R¹ représente le reste d'un acide gras supérieur, linéaire ou ramifié, comportant de 7 à 19 atomes de carbone, et R² représente une chaîne hydrocarbonée ramifiée contenant de 3 à 20 atomes de carbone, comme par exemple l'huile de Purcellin (octanoate de cétostéaryle), l'isononanoate d'isononyle, les benzoates d'alkyle,
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de diisostéaryle, et les esters du pentaérythritol;
- les alcools gras en C₈ à C₂₆ comme l'alcool oléique; et
- leurs mélanges.

Parmi les huiles apolaires, on peut citer:
- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthiocones, les phényl diméthicones, les phényl triméthylsiloxy diphénylsiloxanes, les diphényldiméthicones, les diphényl méthyldiphényl trisiloxanes, les 2-phényléthyl triméthylsiloxysilicates,
- les hydrocarbures ou les hydrocarbures fluorés, linéaires ou ramifiés d'origine synthétique ou minérale, comme les huiles de paraffine (par exemple les isoparaffines), et les hydrocarbures aliphatiques (par exemple l'isododécane), et leurs dérivés, la vaseline, les polydécènes, le polyisobutène hydrogéné tel que le parléam, le squalane, et leurs mélanges.

Par phase grasse solide au sens de la demande, on entend un composé gras lipophile, solide à température ambiante (25°C), ayant une température de fusion supérieure à 40°C et pouvant aller jusqu'à 200°C, en d'autres termes une cire.

Les cires sont celles généralement utilisées dans les domaines cosmétique ou dermatologique. Elles sont notamment d'origine naturelle comme la cire d'abeilles, la cire de Carnauba, de Candellila, d'Ouricoury, du Japon, de fibres de liège ou de canne à sucre, les cires de paraffines, de lignite, les cires microscristallines, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par la synthèse de Fischer-Tropsch, les esters d'acides gras et les glycérides concrets à 40°C, les cires de silicone comme les alkyles, alcoxy et/ou esters de poly(di)méthylsiloxane solide à 40°C.

De façon connue, les compositions utilisées dans le procédé selon l'invention peuvent également contenir les adjuvants habituellement utilisés dans le domaine cosmétique tels que l'eau éventuellement épaissie ou gélifiée par un épaississant ou par un gélifiant de phase aqueuse, les huiles essentielles, les neutralisants, les polymères liposolubles, les actifs cosmétiques ou dermatologiques comme par exemple des émollients, les hydratants, les vitamines, les acides gras essentiels, les filtres solaires, les émulsionnants et les coémulsionnants, les gélifiants hydrophiles ou lipophiles, les additifs hydrophiles ou lipophiles, les conservateurs, les antioxydants, les solvants, les parfums, les charges, les filtres, les absorbeurs d'odeur, les matières colorantes et leurs mélanges.

Les quantités de ces différents adjuvants sont celles classiquement utilisées dans le domaine cosmétique, et par exemple de 0,01% à 10% du poids total de la composition.

Ces adjuvants peuvent, selon leur nature, être introduits dans la phase grasse, dans la phase aqueuse et/ou dans les sphérules lipidiques.

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables selon l'invention, on peut citer les alcools inférieurs, notamment l'éthanol et l'isopropanol, le propylène glycol.

Comme gélifiants hydrophiles selon l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles.

Comme gélifiants lipophiles, on peut citer les argiles modifiées comme les bentones, les sels métalliques d'acides gras comme les stéarates d'aluminium et la silice hydrophobe, éthylcellulose, polyéthylène.

Les compositions utilisées dans le procédé selon l'invention peuvent contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Les compositions utilisées dans le procédé selon l'invention peuvent également associer au moins un agent chélateur d'ions à d'autres agents actifs destinés notamment à la rétention et/ou au traitement des affections cutanées.

Parmi ces agents actifs, on peut citer à titre d'exemple:
- les agents modifiant la différentiation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone,
- les antibactériens tels que le phosphate de clindamycine, l'érythromycine ou les antibiotiques de la classe des tétracyclines;
- les antiparasitaires, en particulier le métronidazole, le crotamiton ou les pyréthrinoïdes;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox;
- les agents antiviraux tels que l'acyclovir;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicyclique, l'acétaminophène ou l'acide glycyrrhétinique;
- les agents anesthésiques tels que le chlorhydrate de lidocaïne et ses dérivés;
- les agents antiprurigineux comme la thénaldine, la triméprazine ou la cyproheptadine;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicyclique, l'acide citrique et de manière générale les acides de fruits, et l'acide n-octanoyl-5-salicyclique;
- les agents anti-radicaux libres, tels que l'alpha-tocophérol ou ses esters, les superoxydes dismutases, certains chélateurs de métaux ou l'acide ascorbique et ses esters;
- les anti-séborrhéiques tels que la prosgestérone;
- les antipelliculaires comme l'octopirox ou la pyrithione de zinc;
- les anti-acnéiques comme l'acide rétinoïque ou le peroxyde de benzoyle.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges Cette matière colorante est généralement présente à raison de 0,01 à 40% du poids total de la composition, de préférence de 5 à 25%.

Les colorants liposolubles sont, par exemple, le rouge Soudan, le DC Red 17, le DC Green 6, le carotène, l'huile de soja, le brun Soudan, le DC Yellow 11, le DC Violet 2, le DC orange 5, le jaune quinoléine.

Les pigments peuvent être blancs ou colorés, minéraux et/ou organiques, enrobés ou non.

On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Les pigments minéraux préférés sont les oxydes de fer, notamment l'oxyde de fer rouge, l'oxyde de fer jaune, l'oxyde de fer rouge et jaune, l'oxyde de fer brun, l'oxyde de fer noir et le dioxyde de titane.

Parmi les pigments organiques, on peut citer:
- le noir de carbone,
- les pigments de type D&C, tel que le D&C Red No 36, et
- les laques à base de carmin de cochenille, de baryum, de strontium, de calcium telle que le D&C Red No 7 calcium lake, d'aluminium, telles que le D&C Red No 27 aluminium lake, le D&C Red No 21 aluminium lake, le FD&C Yellow No 5 aluminium lake, le FD&C Yellow No 6 aluminium lake, le D&C Red No 7 et le FD&D Blue No 1.

Les pigments nacrés peuvent être choisis parmi les pigments nacrés blancs, tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité, ainsi que les pigments nacrés à base d'oxychlorure de bismuth.

Les compositions utilisées dans le procédé selon l'invention peuvent être appliquées sur la peau (sur toute zone cutanée du corps), sur le cuir chevelu, ou sur les muqueuses (buccales, jugale, gingivale et conjonctive).

Selon le mode d'administration, les compositions utilisées dans le procédé selon l'invention peuvent se présenter sous toutes les formes galéniques normalement utilisées.

Pour une application topique sur la peau, ces compositions peuvent avoir la forme de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ionique. Ces compositions sont préparées selon les méthodes usuelles.

Les compositions utilisées dans le procédé selon l'invention peuvent être également appliquées sur pour le cuir chevelu sous forme de solutions aqueuses, alcooliques ou hydroalcooliques, ou sous forme de crèmes, de gels, d'émulsions, de mousses ou encore sous forme de compositions pour aérosols comprenant également un agent propulseur sous pression.

Les quantités des différents constituants des compositions utilisées dans le procédé selon l'invention sont celles classiquement utilisées dans les domaines considérés.

Les compositions utilisées dans le procédé selon l'invention constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes, comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions utilisées dans le procédé selon l'invention peuvent également consister en des préparations solides constituant des savons ou des pains de nettoyage.

Les compositions utilisées dans le procédé selon l'invention peuvent aussi être conditionnées sous forme de composition pour aérosol comprenant également un agent propulseur sous pression.

Les compositions utilisées dans le procédé selon l'invention peuvent aussi être des compositions pour soins capillaires, et notamment un shampooing, une lotion de mise en plis, une lotion traitante, une crème ou un gel coiffant, une composition pour teintures (notamment teintures d'oxydation) éventuellement sous forme de shampooings colorants, des lotions restructurantes pour les cheveux, une composition de permanente (notamment une composition pour le premier temps d'une permanente), une lotion ou un gel anti-chute, un shampooing anti-parasitaire, etc.

Les compositions utilisées dans le procédé selon l'invention peuvent aussi être à usage bucco-dentaire, par exemple une pâte dentifrice. Dans ce cas, les compositions peuvent contenir des adjuvants et additifs usuels pour les compositions à usage buccal, et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Les quantités des différents constituants des compositions selon l'invention sont celles classiquement utilisées dans les domaines considérés.

Les procédés selon l'invention peuvent être mis en oeuvre notamment en appliquant les compositions hygiéniques ou cosmétiques telles que définies précédemment, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions, les proportions indiquées sauf indication contraire sont des pourcentages en poids.

### EXEMPLE

### Effet de l'EDTA sur la réactivité cutanée stimulée par l'acide lactique chez les sujets à peau sensible

Un test fonctionnel *in vivo* est réalisé pour mettre en évidence, sur une population à peau sensible, les effets apaisants en prétraitement (monoapplication) d'un agent chélateur (EDTA) vis à vis des sensations de picotements induits par l'application d'une solution (ou Stinger) d'acide lactique sur les sillons nasogéniens.

### Sujets

- 12 volontaires sains, de sexe féminin, âgés de 18 à 45 ans de phototype I à IV à peau sensible,

### Produits

- agent chélateur : sel de disodium éthylènediamine tétraacétate (EDTA),
- solution à 10% d'acide lactique.
- composition cosmétique selon l'invention CM₁ contenant 2% d'EDTA et se présentant sous forme de gel,
- composition cosmétique CM₀ ne contenant pas d'EDTA (véhicule de CM₁) et se présentant sous forme de gel.

Les compositions CM₁ et CM₀ sont présentées dans le tableau 1 ci-dessous.

**TABLEAU 1**

| | Composition CM₁ | Composition CM₀ |
|---|---|---|
| méthyl paraben | 0,2% | 0,2% |
| EDTA disodium | 2% | 0% |
| triéthalonamine | 0,7% | 0,7% |
| carbomer | 0,7% | 0,7% |
| eau | 96,4% | 98,4% |

### Méthodologie

II s'agit d'une étude prospective, monocentrique, en double-aveugle, randomisée, contrôlée versus véhicule, avec une comparaison intra-individuelle (sillon droit/sillon gauche).

On applique à t=0 la composition cosmétique CM₀ sur l'un des sillons nasogéniens , et la composition CM₁ sur l'autre sillon.

Puis on applique à t=30 minutes, une solution à 10% d'acide lactique sur les deux zones prétraitées, l'une avec la composition CM₀ et l'autre avec la composition CM₁.

On évalue ensuite, entre t=30 minutes et t=35 minutes les sensations de picotements ressenties par le sujet en utilisant l'échelle suivante:
0 = pas de sensation
1 = léger ou douteux
2 = modéré
3 = important

### Résultats

Les résultats sont présentés en terme de moyenne de score de picotements tous instants confondus, et sont rassemblés dans le tableau 2.

Pour vérifier que les résultats obtenus sont statistiquement significatifs, on établit l'hypothèse qu'il n'y a pas de différence entre les effets respectifs des compositions CM₁ et CM₀. Puis, on calcule la probabilité p pour que cette hypothèse soit vérifiée lors du test de Wilcoxon sur les données appariées. Lorsque p est inférieur ou égal à 0,05, le résultat (score moyen de picotements) est statistiquement significatif.

**TABLEAU 2**

| | Score moyen de picotements ± écart-type de la moyenne | valeur de p* Test de Wilcoxon sur données appariées | Comparaison CM₁/CM₀ |
|---|---|---|---|
| CM₀ | 1,53 ± 0,18 | 0,0234 | diminution de 41% |
| (0% d'EDTA) | | | |
| CM₁ | 0,90 ± 0,1 | | |
| (2% d'EDTA) | | | |

On observe une diminution statistiquement significative du score de picotements lors d'une application préalable de la composition cosmétique CM₁ à 2% d'EDTA.

## Revendications

1. Utilisation cosmétique topique d'une quantité effective d'au moins un agent chélateur d'ions pour augmenter le seuil de tolérance de la peau sensible ou intolérante, dans une composition cosmétique et/ou de l'hygiène.

2. Utilisation topique d'une quantité effective d'au moins un agent chélateur d'ions pour la fabrication d'une composition dermatologique destinée à augmenter le seuil de tolérance de la peau sensible ou intolérante.

3. Utilisation selon la revendication 1 ou 2 **caractérisée en ce que** la composition est une composition de pré-traitement.

4. Utilisation selon l'une quelconque des revendications précédentes **caractérisée en ce que** l'agent chélateur est un agent chélateur chimique choisi parmi :
- l'acide aminotriméthyle phosphonique,
- l'acide β-alanine diacétique,
- l'acide citrique,
- la cyclodextrine,
- l'acide cyclohexanediamine tétracétique,
- l'acide diéthylènetriamine pentaméthylène phosphonique,
- l'acide diéthanolamine N-acétique (diéthanolamine N-acétique),
- l'acide éthylène diamine tétracétique (EDTA ou YH₄) et ses sels de sodium (YH₃Na, Y₂H₂Na₂, YHNa₃ et YNa₄), de potassium (YH₃K, Y₂H₃K₃ et YK₄), de calcium disodium, de diammonium et ses sels de triéthanolamine (TEA-EDTA),
- l'acide étidronique,
- l'acide galactanique,
- l'acide hydroxyéthyl éthylènediamine tétracétique (HEDTA) et son sel trisodique,
- l'acide gluconique,
- l'acide glucuronique,
- l'acide nitrilotriacétique (NTA) et son sel trisodique,
- l'acide pentétique,
- l'acide phytique,
- l'acide ribonique,
- le citrate de diammonium,
- l'azacycloheptane diphosphonate de disodium,
- le pyrophoshate de disodium,
- l'hydroxypropyl cyclodextrine,
- le méthyl cyclodextrine,
- le triphosphate de pentapotassium,
- l'aminotriméthylène phosphonate de pentasodium,
- l'éthylènediamine tétraméthylène phosphonate de pentasodium,
- le pentétate de pentasodium,
- le triphosphate de pentasodium,
- le citrate de potassium,
- EDTMP de potassium,
- EDTMP de sodium,
- le chitosan méthylène phosphonate de sodium,
- l'hexamétaphosphate de sodium,
- le métaphosphate de sodium,
- le polyphosphate de potassium,
- le polyphosphate de sodium,
- le trimétaphosphate de sodium,
- le dihydroxyéthylglycinate de sodium,
- le gluconate de potassium,
- le gluconate de sodium,
- le glucopeptate de sodium,
- le glycereth-1 polyphosphate de sodium,
- le pyrophosphate de tétrapotassium,
- le polyphosphate de triéthanolamine (TEA),
- le pyrophosphate de tétrasodium,
- le phosphate de trisodium.
- l'oxyde triphosphonométhylamine de potassium,
- le métasilicate de sodium,
- le phytate de sodium,
- le polydiméthylglycinophénolsulfonate de sodium,
- le tétrahydroxyéthyl éthylène diamine,
- le tétrahydroxypropyl éthylène diamine,
- l'étidronate de tétrapotassium,
- l'étidronate de tétrasodium,
- l'iminodisuccinate de tétrasodium,
- l'éthylènediamine disuccinate de trisodium,
- l'acide éthanolamine N,N-diacétique,
- l'acétate de disodium,
- le dimercaprol,
- la déferoxamine,
- le Zylox.

5. Utilisation selon la revendication 4, **caractérisé en ce que** l'agent chélàteur est un chélateur chimique, choisi parmi l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'éthylènediamine disuccinate de trisodium, et leurs mélanges.

6. Utilisation selon l'une quelconque des revendications 1 à 3, **caractérisé en ce que** l'agent chélateur d'ion est un chélateur biologique choisi parmi la métallothionéine, la transférine, la calmoduline, le chitosan méthylène phosphonate, et leurs mélanges.

7. Utilisation selon l'une quelconque des revendications précédentes, **caractérisé en ce que** les ions chélates sont préférentiellement les ions Na⁺, K⁺, Ca²⁺ et Cl⁻.

8. Utilisation selon l'une quelconque des revendications précédentes, **caractérisé en ce que** l'agent chélateur est présent dans la composition en une quantité représentant de 10⁻⁶ à 10% en poids, de préférence en une quantité représentant de 0,1 à 5% en poids, et mieux environ 2% en poids du poids total de la composition.

9. Utilisation selon l'une quelconque des revendications précédentes, **caractérisé en ce que** la composition comprend au moins une phase grasse liquide ou solide.

10. Utilisation selon la revendication 9, **caractérisé en ce que** la phase grasse est liquide et contient au moins une huile choisie parmi les huiles hydrocarbonées végétales, animales, de synthèse, les huiles siliconées et les huiles fluorées.

11. Utilisation selon la revendication 9, **caractérisé en ce que** la phase grasse est solide et contient au moins une cire choisie parmi les cires d'origine naturelles, telles que la cire d'abeilles, de carnauba, de paraffine, les esters d'acides gras, les alcools gras et les cires siliconées.

12. Utilisation selon l'une quelconque des revendications précédentes, **caractérisé en ce que** la composition contient au moins un actif cosmétique.

13. Utilisation selon l'une quelconque des revendications précédentes, **caractérisé en ce que** la composition contient au moins un additif choisi parmi l'eau, les antioxydants, les huiles essentielles, les conservateurs, les neutralisants, les polymères liposolubles, les charges, les parfums, les émulsionnants, les gélifiants, les filtres, les absorbeurs d'odeur et les matières colorantes.

14. Utilisation selon l'une quelconque des revendications précédentes, **caractérisé en ce que** la composition se présente sous forme de solution aqueuse ou huileuse ou de dispersion du type lotion ou sérum pour une application topique sur la peau.

15. Utilisation selon l'une quelconque des revendications 1 à 13, **caractérisé en ce que** la composition se présente sous forme d'une émulsion de consistance liquide ou semi-liquide du type lait, obtenue par dispersive d'une phase grasse dans une phase aqueuse (H/E) ou inversement (E/H), pour une application topique sur la peau.

16. Utilisation selon l'une quelconque des revendications 1 à 13, **caractérisé en ce que** la composition se présente sous forme de suspension ou d'émulsion de consistance molle du type crème ou gel aqueux ou anhydre, pour une application topique sur la peau.

17. Utilisation selon l'une quelconque des revendications 1 à 13, **caractérisé en ce que** la composition se présente sous forme de solution aqueuse alcoolique ou hydroalcoolique pour une application sur le cuir chevelu.

18. Utilisation selon l'une quelconque des revendications 1 à 13, **caractérisé en ce que** la composition se présente sous forme de crème, de gel d'émulsion ou de mousse, pour une application sur le cuir chevelu.

19. Utilisation selon l'une quelconque des revendications 1 à 10, **caractérisé en ce que** la composition est conditionnée sous forme d'aérosol et comprend également un agent propulseur sous pression, pour une application sur le cuir chevelu.

## Claims

1. Topical cosmetic use of an effective amount of at least an ion chelating agent for increasing the tolerance threshold of sensitive or intolerant skin in a cosmetic and/or hygienic composition.

2. Topical use of an effective amount of at least an ion chelating agent for the making of a dermatological composition for increasing the tolerance threshold of sensitive or intolerant skin.

3. Use according to claim 1 or 2, **characterized in that** the composition is a pre-treating composition.

4. Use according to any one of the preceding claims, **characterized in that** the chelating agent is a chemical chelating agent selected among:
- aminotriemethyl phosphonic acid,
- β-alamine diacetric acid,
- citric acid,
- cyclodextrine,
- cyclohexanediamine tetraacetic acid,
- diethylenetriamine pentanethylene phosphonic acid,
- diethanolamine N-acetic (diethanolamine N acetic) acid,
- ethylene diamine tetraacetic acid (EDTA or YH₄) and sodium salts thereof (YH₃Na, Y₂H₂Na₂, YHNa₃ and YNa₄) and potassium salts thereof (YH₃K, Y₂H₃K₃ and YK₄) disodium calcium salt thereof, diammonium salt thereof and triethanolamine salts thereof (TEA-EDTA),
- etidronic acid,
- galactanic acid,
- hydroxyethyl ethylene diamine tetraacetic acid (HEDTA) and the trisodium salt thereof,
- gluconic acid,
- glucuronic acid,
- nitrilotriacetic acid (NTA) and the trisodium salt thereof,
- pentetic acid,
- phytic acid,
- ribonic acid,
- diammonium citrate,
- azacycloheptane disodium diphosphonate,
- disodium pyrophosphate,
- hydroxypropyl cyclodextrine,
- methyl cyclodextrine,
- pentapotassium triphosphate,
- aminotrimethylene pentasodium phosphonate,
- ethylene diamine tetramethylene pentasodium phosphonate,
- pentasodium pentetate,
- pentasodium triphosphate,
- potassium citrate,
- potassium EDTMP,
- sodium EDTMP,
- chitosan methylene sodium phosphonate,
- sodium hexametaphosphate,
- sodium metaphosphate,
- potassium polyphosphate,
- sodium polyphosphate,
- sodium trimetaphosphate,
- sodium dihydroxyethylglycinate,
- potassium gluconate,
- sodium gluconate,
- sodium glucopeptate,
- glycereth-1 sodium polyphosphate,
- tetrapotassium pyrophosphate,
- triethanolamine polyphosphate (TEA),
- tetrasodium pyrophosphate,
- trisodium phosphate,
- potassium triphosphonomethylamine oxyde,
- sodium metasilicate,
- sodium phytate,
- sodium polydimethylglycinophenolsulfonate,
- tetrahydroxyethyl ethylene diamine,
- tetrahydroxypropyl ethylene diamine,
- tetrapotassium etidronate,
- tetrasodium iminodisuccinate,
- trisodium ethylenediamine disuccinate,
- ethanolamine N,N diacetic acid,
- disodium acetate,
- dimercapnol,
- deferoxamine,
- zylox.

5. Use according to claim 4, **characterized in that** the chelating agent is a chemical chelating agent, selected among ethylenediamine tetracetic acid (EDTA) and its sodium, potassium, calcium disodium, diammonium, and triethanolamine salts (TEA-EDTA), hydroxyethyl ethylenediamine tetracetic acid (HEDTA) and its trisodium salt.

6. Use according to any one of claims 1 to 3, **characterized in that** the ion chelating agent is a biological chelating agent selected among metallothionein, transferin, calmodulin, chitosan methylene phosphonate, and mixtures thereof.

7. Use according to any one of the preceding claims, **characterized in that** the chelating ions are preferably the Na⁺, K⁺, Ca²⁺ and Cl⁻ ions.

8. Use according to any one of the preceding claims **characterized in that** the chelating agent is present in the composition in a quantity representing from 10⁻⁶ to 10% by weight, preferably from 0.1 to 5% by weight, and more preferably about 2% by weight of the total weight of the composition.

9. Use according to any one of the preceding claims **characterized in that** the composition comprises at least one liquid or solid fatty phase.

10. Use according to claim 9, **characterized in that** the fatty phase is liquid and comprises at least an oil selected among synthetic, animal, plant hydrocarbon oils, silicone oils and fluorinated oils.

11. Use according to claim 9, **characterized in that** the fatty phase is solid and comprises at least a wax selected among the natural waxes such as beeswax, Carnauba wax, paraffin wax, the esters of fatty acids, the fatty alcohols and the silicone waxes.

12. Use according to any one of the preceding claims, **characterized in that** the composition comprises at least a cosmetic active ingredient.

13. Use according to any one of the preceding claims, **characterized in that** the composition comprises at least one additive selected among water, the antioxidants, the essential oils, the preservatives, the neutralizing agents, the liposoluble polymers, the fillers, the perfumes, the emulsifying agents, the gelling agents, the filters, the odour absorbers and the colorants.

14. Use according to any one of the preceding claims, **characterized in that** the composition is as an aqueous or oily solution or dispersion of the lotion or serum type, for topical application to the skin.

15. Use according to any one of claims 1 to 13, **characterized in that** the composition is as an emulsion of liquid or semi-liquid consistency of the milk type, obtained by dispersion of a fatty phase in an aqueous phase (O/W) or of an aqueous phase in a fatty phase (W/O), for topical application to the skin.

16. Use according to any one of claims 1 to 13, **characterized in that** the composition is as a suspension or emulsion of soft consistency of the cream or aqueous or anhydrous gel type, for topical application to the skin.

17. Use according to any one of claims 1 to 13, **characterized in that** the composition is as an aqueous, alcoholic or hydroalcoholic solution, for application to the scalp.

18. Use according to any one of claims 1 to 13, **characterized in that** the composition is as a cream, gel, emulsion or foam, for application to the scalp.

19. Use according to any one of claims 1 to 10, **characterized in that** the composition is packaged as an aerosol and also comprises a propellant agent under pressure, for application to the scalp.

## Patentansprüche

1. Topische kosmetische Verwendung einer wirksamen Menge wenigstens eines Chelatbildners für Ionen, um die Toleranzschwelle von empfindlicher oder intoleranter Haut zu erhöhen, in einer kosmetischen und/oder Hygienezusammensetzung.

2. Topische Verwendung einer wirksamen Menge wenigstens eines Chelatbildners für Ionen zur Herstellung einer dermatologischen Zusammensetzung, die dazu bestimmt ist, die Toleranzschwelle der empfindlichen oder intoleranten Haut zu erhöhen.

3. Verwendung nach Anspruch 1 oder 2, **dadurch gekennzeichnet, dass** die Zusammensetzung eine Vorbehandlungszusammensetzung ist.

4. Verwendung nach einem der vorstehenden Ansprüche, **dadurch gekennzeichnet, dass** der Chelatbildner ein chemischer Chelatbildner ist, ausgewählt aus:
- Aminotrimethylphosphonsäure,
- β-Alanindiessigsäure,
- Citronensäure,
- Cyclodextrin,
- Cyclohexandiamintetraessigsäure,
- Diethylentriaminpentamethylenphosphonsäure,
- Diethanolamin-N-essigsäure (Diethanolamin-N-acetat),
- Ethylendiamintetraessigsäure (EDTA oder YH₄) und ihre Natriumsalze (YH₃Na, Y₂H₂Na₂, YHNa₃ und YNa₄) und Kaliumsalze (YH₃K, Y₂H₃K₃ und YK₄), ihre Calciumdinatriumsalze, ihre Ammoniumsalze und ihre Triethanolaminsalze (TEA-EDTA),
- Etidronsäure,
- Galactansäure,
- Hydroxyethylethylendiamintetraessigsäure (HEDTA) und ihr Trinatriumsalz,
- Gluconsäure,
- Glucuronsäure,
- Nitrilotriessigsäure (NTA) und ihr Trinatriumsalz,
- Diethylentriaminpentaessigsäure,
- Phytinsäure,
- Ribonsäure,
- Diammoniumcitrat,
- Azacycloheptandiphosphonat-Dinatrium,
- Dinatriumpyrophosphat,
- Hydroxypropylcyclodextrin,
- Methylcyclodextrin,
- Pentacaliumtriphosphat,
- Aminotrimethylenphosphonat-Pentanatrium,
- Ethylendiamintetramethylenphosphonat-Pentanatrium,
- Diethylentriaminpentaacetat-Pentanatrium,
- Pentanatriumtriphosphat,
- Kaliumcitrat,
- Kalium-EDTMP,
- Natrium-EDTMP,
- Natriumchitosanmethylenphosphonat,
- Natriumhexametaphosphat,
- Natriummetaphosphat,
- Kaliumpolyphosphat,
- Natriumpolyphosphat,
- Natriumtrimetaphosphat,
- Natriumdihydroxyethylglycinat,
- Kaliumgluconat,
- Natriumgluconat,
- Natriumglucopeptat,
- Glycereth-1-polyphosphat-Natrium,
- Tetrakaliumpyrophosphat,
- Triethanolaminpolyphosphat bzw. TEA-Polyphosphat,
- Tetranatriumpyrophosphat,
- Trinatriumphosphat,
- Kaliumtriphosphonomethylaminoxid,
- Natriummetasilicat,
- Natriumphytat,
- Natriumpolydimethylglycinophenolsulfonat,
- Tetrahydroxyethylethylendiamin,
- Tetrahydroxypropylethylendiamin,
- Tetrakaliumetidronat,
- Tetranatriumetidronat,
- Tetranatriumiminodisuccinat,
- Trinatriumethylendiamindisuccinat,
- Ethanolamin-N, N-diessigsäure,
- Dinatriumacetat,
- Dimercaprol,
- Deferoxamin,
- Zylox.

5. Verwendung nach Anspruch 4, **dadurch gekennzeichnet, dass** der Chelatbildner ein chemischer Chelatbildner ist, der aus Ethylendiamintetraessigsäure (EDTA) und ihren Natrium-, Kalium-, Calciumdinatrium-, Diammoniumsalzen, ihrem Triethanolaminsalz (TEA-EDTA), Hydroxyethylethylendiamintetraessigsäure (HEDTA) und ihrem Trinatriumsalz, Trinatriumethylendiamindisuccinat und ihren Gemischen ausgewählt ist.

6. Verwendung nach einem der Ansprüche 1 bis 3, **dadurch gekennzeichnet, dass** der Chelatbildner für Ionen ein biologischer Chelatbildner ist, der aus Metallothionein, Transferrin, Calmodulin, Chitosanmethylenphosphonat und deren Gemischen ausgewählt ist.

7. Verwendung nach einem der vorangehenden Ansprüche, **dadurch gekennzeichnet, dass** die Chelationen vorzugsweise die Ionen Na⁺, K⁺, Ca²⁺ und Cl⁻ sind.

8. Verwendung nach einem der vorangehenden Ansprüche, **dadurch gekennzeichnet, dass** der Chelatbildner in der Zusammensetzung in einer Menge vorliegt, die 10⁻⁶ bis 10 Gew.-% darstellt, vorzugsweise in einer Menge vorliegt, die 0,1 bis 5 Gew.-% darstellt und am besten etwa 2 Gew.-% des Gesamtgewichts der Zusammensetzung darstellt.

9. Verwendung nach einem der vorangehenden Ansprüche, **dadurch gekennzeichnet, dass** die Zusammensetzung wenigstens eine flüssige oder feste Fettphase umfasst.

10. Verwendung nach Anspruch 9, **dadurch gekennzeichnet, dass** die Fettphase flüssig ist und wenigstens ein Öl enthält, das aus den pflanzlichen, tierischen, synthetischen Kohlenwasserstoffölen, den Siliconölen und den fluorierten Ölen ausgewählt ist.

11. Verwendung nach Anspruch 9, **dadurch gekennzeich-net, dass** die Fettphase fest ist und wenigstens ein Wachs enthält, das unter Wachsen natürlicher Herkunft wie Bienenwachs, Carnaubawachs, Paraffinwachs, Fettsäureestern, Fettalkoholen und Siliconwachsen ausgewählt ist.

12. Verwendung nach einem der vorangehenden Ansprüche, **dadurch gekennzeichnet, dass** die Zusammensetzung wenigstens einen kosmetischen Wirkstoff enthält.

13. Verwendung nach einem der vorangehenden Ansprüche, **dadurch gekennzeichnet, dass** die Zusammensetzung wenigstens ein Additiv enthält, das aus Wasser, Antioxydantien, ätherischen Ölen, Konservierungsmitteln, Neutralisiermitteln, fettlöslichen Polymeren, Füllstoffen, Parfüms, Emulgiermitteln, Geliermitteln, Filtern, Absorptionsmitteln für Geruch und Färbemitteln ausgewählt ist.

14. Verwendung nach einem der vorangehenden Ansprüche, **dadurch gekennzeichnet, dass** die Zusammensetzung in Form einer wässrigen oder öligen Lösung oder Dispersion des Lotion- oder Serumtyps für eine topische Anwendung auf die Haut vorliegt.

15. Verwendung nach einem der Ansprüche 1 bis 13, **dadurch gekennzeichnet, dass** die Zusammensetzung in Form einer Emulsion mit flüssiger oder halbflüssiger Konsistenz des Milchtyps, die durch Dispersion einer Fettphase in einer wässrigen Phase (Ö/W) oder umgekehrt (W/Ö) erhalten wird, für eine topische Anwendung auf die Haut vorliegt.

16. Verwendung nach einem der Ansprüche 1 bis 13, **dadurch gekennzeichnet, dass** die Zusammensetzung in Form einer Suspension oder Emulsion mit weicher Konsistenz des Creme- oder Geltyps, wässrig oder wasserfrei, für eine topische Anwendung auf die Haut vorliegt.

17. Verwendung nach einem der Ansprüche 1 bis 13, **dadurch gekennzeichnet, dass** die Zusammensetzung in Form einer wässrig-alkoholischen oder hydroalkoholischen Lösung für eine Anwendung auf die behaarte Kopfhaut vorliegt.

18. Verwendung nach einem der Ansprüche 1 bis 13, **dadurch gekennzeichnet, dass** die Zusammensetzung in Form einer Creme, eines Emulsionsgels oder eines Schaums für die Anwendung auf die behaarte Kopfhaut vorliegt.

19. Verwendung nach einem der Ansprüche 1 bis 10, **dadurch gekennzeichnet, dass** die Zusammensetzung in Aerosolform konditioniert ist und auch ein Treibmittel unter Druck umfasst, für eine Anwendung auf die behaarte Kopfhaut.
